# EUROPEAN PATENT APPLICATION

(11) **EP 4 518 097 A1**
(43) Date of publication of application: **05.03.2025**
(21) Application number: 23195099.9
(22) Date of filing: 04.09.2023
(51) Int. Cl.: H02J 50/12, H03K 17/284, H03K 17/30

(54) **A CHARGING CIRCUITRY, AN IMPLANT DEVICE, AND A METHOD FOR RECEIVING POWER THROUGH WIRELESS POWER TRANSFER**

(71) Applicant: Stichting IMEC Nederland, 5656 AE Eindhoven (NL)
(72) Inventor: PASTERKAMP, Jesse, 5616 SC Eindhoven (NL); STANZIONE, Stefano, 5508 AH Veldhoven (NL); GARRIPOLI, Carmine, 5654 NW Eindhoven (NL)
(74) Representative: AWA Sweden AB

(57) **Abstract**

A charging circuitry (100; 200) for receiving power through wireless power transfer comprises: a power receiver (110; 210) configured to receive an alternating current (AC) wireless power signal and store energy through resonance; a rectifying switch (120; 220) configured to selectively connect an output (116; 216) of the power receiver (110; 210) to a load (130; 230); a comparator (140; 240) connected to the output (116; 216) of the power receiver (110; 210) and configured to identify an extreme point of an output voltage signal (*vₛ*) of the power receiver (110; 210) and configured to cause the rectifying switch (120; 220) to be turned on based on identification of the extreme point; and a delay circuit (170; 270) configured to control a state of the comparator such that the comparator (140; 240) is disabled for a delay period between successive instances of the rectifying switch (120; 220) being turned on.

## Description

### Technical field

The present description relates to wireless power transfer. In particular, the present description relates to a charging circuitry and a method for receiving power through wireless power transfer.

### Background

Power transfer may be used in various applications in order to provide power to a power receiving device such that the power receiving device remains operational.

Wireless powering by means of the powering apparatus is useful in numerous applications. By using wireless powering, a wire between the powering apparatus and a power receiving device is not needed so as to provide a convenient or practical manner of powering the power receiving device. Also, in some instances, it is not possible to use a wire for powering, such as when the power receiving device is arranged in a body of a person as an implanted or ingested device. Then, wireless powering can be used in order to enable a prolonged lifetime of the implanted or ingested device.

Wireless powering by means of inductive coupling uses loops, or coils, to transfer energy via a magnetic field from the powering apparatus to the power receiving device. However, the power received by the power receiving device may be very low in certain operating conditions, for instance if the location of the implanted or ingested device corresponds to a substantial distance between the powering apparatus and the implanted or ingested device.

If the power receiving device generates an alternating current (AC) voltage based on the received signal, a rectifier is needed to obtain a direct current (DC) voltage suitable for up-conversion. The power receiving device may be configured to store energy from multiple resonant cycles of the received signal. However, if the power of the received signal is weak, the number of cycles during which energy is stored increases and a static power consumption for controlling the rectifier limits overall efficiency of powering of the power receiving device.

Thus, there is a need for efficient powering of a power receiving device.

### Summary

An objective of the present description is to provide control of wireless power transfer, such that efficient power transfer is provided. A particular objective of the present description is to provide a charging circuitry which efficiently receives power through wireless power transfer.

These and other objectives are at least partly met by the invention as defined in the independent claims. Preferred embodiments are set out in the dependent claims.

According to a first aspect, there is provided a charging circuitry for receiving power through wireless power transfer, said charging circuitry comprising: a power receiver configured to receive an alternating current (AC) wireless power signal and configured to store energy through resonance; a rectifying switch configured to selectively connect an output of the power receiver to a load; a comparator connected to the output of the power receiver and configured to identify an extreme point of an output voltage signal of the power receiver and configured to cause the rectifying switch to be turned on based on identification of the extreme point of the output voltage signal; and a delay circuit configured to control a state of the comparator such that the comparator is disabled for a delay period between successive instances of the rectifying switch being turned on.

The charging circuitry allows controlling the rectifying switch such that the rectifying switch may be turned on synchronously with an extreme point of the output voltage signal of the power receiver. The charging circuitry further comprises a delay circuit which ensures that the comparator may be disabled for a delay period. The delay circuit may operate independently, that is non-synchronously, with the output voltage signal of the power receiver. Thus, the comparator need not be a continuous-time comparator, as is required for a synchronous control of the rectifying switch. This implies that the power consumption of the charging circuitry may be scaled down in comparison to the use of a continuous-time comparator with a number of resonant cycles of the output voltage signal corresponding to the delay period.

In other words, thanks to the comparator being operated intermittently, as controlled by the delay circuit, the charging circuitry is configured to efficiently receiving power through wireless power transfer.

The power receiver may comprise a resonant circuit defining a resonant frequency. For instance, the power receiver may comprise an inductor (L) and a capacitor (C), defining an LC circuit having a resonant frequency defined by the inductance and capacitance of the inductor and the capacitor, respectively. The resonant circuit may be configured to store energy by providing the output voltage signal, which may have a frequency corresponding to the resonant frequency of the power receiver. The amplitude of the output voltage signal may increase for each cycle of the resonant frequency as long as energy is stored and not extracted from the power receiver.

The power receiver may be configured to receive power through inductive wireless power transfer. However, it should be realized that the power receiver may store energy through resonance in another manner such that the wireless power transfer need not necessarily be performed by inductive wireless power transfer. For instance, the power receiver may alternatively be configured to receive power through capacitive resonant wireless power transfer.

The rectifying switch may be any switch that allows control of connecting the output from the power receiver to the load. As an example, the rectifying switch may be a transistor which may be controlled by a control signal applied to a gate of the transistor for opening or closing a connection between the output from the power receiver and the load.

The comparator may be configured to identify a positive or negative maximum. For instance, the comparator may be configured to identify a positive maximum, or a peak, of the output voltage signal.

It should further be realized that the comparator need not necessarily detect an exact point in time of the absolute extreme point. Rather, it should be realized that the extreme point may be identified by identifying that the output voltage signal is larger than (or, for a minimum of the output voltage signal, smaller than) a threshold such that it may be determined that the output voltage signal is in a region around the extreme point.

It should be realized that the comparator may be implemented in various manners. For instance, the comparator may be configured to identify the extreme point of the output voltage signal by identifying a value of the output voltage signal indicative of the extreme point, i.e., by comparing the output voltage signal to a threshold. However, the comparator may not need to provide comparison based on an absolute value of the output voltage signal. For instance, the output voltage signal may alternatively be integrated, which may be used by the comparator to identify the extreme point of the output voltage signal.

The delay circuit is configured to control a state of the comparator so as to control when the comparator is disabled. For instance, the comparator may be normally disabled, and the delay circuit may be configured to provide a control signal so as to enable the comparator when the comparator is to be active to identify the extreme point of the output voltage signal. However, the comparator may alternatively be normally enabled, and the delay circuit may be configured to provide a control signal so as to disable the comparator in order to generate the delay between successive instances of the rectifying switch being turned on.

It should be realized that the delay period is a period of time between successive instances of the rectifying switch being turned on. It should further be realized that the delay period is not necessarily exactly equal to the delay between successive instances of the rectifying switch being turned on. Rather, after the delay period, the comparator may start a process to identify the extreme point of the output voltage signal. The comparator may not immediately identify the extreme point such that some further delay may occur before the rectifying switch is caused to be turned on by the comparator.

The delay circuit may be configured to control the comparator to be disabled for a delay period which is equal to or longer than a period of the output voltage signal. For instance, the delay period may correspond to a number of periods (cycles) of the output voltage signal. It should be realized that the delay period need not necessarily correspond to an integer number of periods of the output voltage signal.

Alternatively, the delay circuit may be configured to control the comparator to be disabled for a delay period which is shorter than a period of the output voltage signal. This implies that the comparator may be configured to cause the rectifying switch to be turned on during each period of the output voltage signal. However, the charging circuitry may still consume less power compared to having a comparator which is continuously processing the output voltage signal such that it may be worthwhile to define a delay period which is a fraction of the period of the output voltage signal.

It should further be realized that the delay period may be programmable such that the delay period may be dynamically changed. This may for instance be used in dependence of a strength of the wireless power signal received by the power receiver.

It should be realized that a duration of the delay period may depend on an application in which the charging circuitry is used. As an example, the delay period may be in a range of 0.2 - 12 times the period of the output voltage signal. If a resonant circuit of the power receiver has a high quality factor, even larger delay periods, such as delay periods up to 20 times the period of the output voltage signal or even longer delay periods may be used.

According to an embodiment, the delay circuit is configured to output a trigger for activating the comparator, wherein the comparator is configured to, upon receiving the trigger, initiate identifying of the extreme point of the output voltage signal of the power receiver.

Thus, the delay circuit may trigger the comparator to control when the comparator is active for identifying the extreme point. The delay circuit does not need to be synchronized with a period of the output voltage signal. Rather, the comparator may identify a next extreme point occurring after the trigger is received.

As discussed above, the trigger may control the comparator that is normally disabled to be enabled when receiving the trigger. Alternatively, the comparator may be normally enabled and may receive a control signal from the delay circuit to ensure that the comparator is disabled for the delay period. Thus, the delay circuit being configured to output a trigger may correspond to the delay circuit ceasing to output a signal for disabling the comparator.

According to an embodiment, the delay circuit is configured to receive an external control signal for controlling the delay period of the delay circuit.

This implies that the charging circuitry may be externally controlled. The external control signal may be provided such that settings of the charging circuitry may be dynamically changed in dependence of the external control signal. For instance, the external control signal may be provided by a power transmitter for providing power to the charging circuitry. According to an alternative, the delay circuit may receive a control signal from a circuit that may estimate power stored in the resonant circuit.

According to an embodiment, the comparator is configured to output an activation signal for turning on the rectifying switch upon the identification of the extreme point of the output voltage signal of the power receiver, wherein the delay circuit is also configured to receive the activation signal for triggering start of a following delay period.

Thus, the rectifying switch may be turned on to connect the output of the power receiver to the load based on receiving the activation signal. The same activation signal may also trigger the delay circuit. Thus, the delay circuit may be controlled based on the same activation signal that is received by the rectifying switch.

The delay circuit may be configured to output a trigger to the comparator, the trigger being output after the delay period has passed from the delay circuit being triggered by the activation signal.

The start of the following delay period may be defined by the activation signal going high (or low) to turn on the rectifying switch. However, the start of the following delay period may alternatively be defined by the activation signal going low (or high) to turn off the rectifying switch after the rectifying switch has been activated.

It should be realized that the start of the following delay period as determined by the delay circuit need not necessarily be triggered by an output signal directly from the comparator. Rather, the delay circuit may be an off-delay circuit and the comparator may output the activation signal to an on-delay circuit. The on-delay circuit may provide an output based on the activation signal to turn on the rectifying switch. Also, the on-delay circuit may define a delay so as to control a period during which the rectifying switch is turned on. The on-delay circuit may thus provide a control to turn off the rectifying switch. The on-delay circuit may also trigger start of the following delay period as determined by the off-delay circuit. Thus, the off-delay circuit may be triggered based on the output of the on-delay circuit causing the rectifying switch to be turned off.

It should further be realized that the comparator may be enabled based on a trigger from a first off-delay circuit. Then, the activation signal provided by the comparator may control start of a delay period of a second off-delay circuit. This may be used for enabling the charging circuitry to provide multiple interleaved delays.

According to an embodiment, the comparator is configured to detect at least one edge of the output voltage signal.

Thus, the comparator may be configured to detect that the output voltage signal changes based on detecting an edge. This may be an efficient manner of identifying that the output voltage signal reaches an extreme point. For instance, a maximum of the output voltage signal may be preceded by a rising edge.

According to an embodiment, the comparator is configured to detect a rising edge and a falling edge of the output voltage signal.

This implies that the comparator may identify edges around an extreme point of the output voltage signal. This implies that the detection of both a rising edge and a falling edge allows the comparator to control a period during which the rectifying switch is turned on, such that the comparator determines when the rectifying switch is to be turned on and when the rectifying switch is to be turned off again.

According to an embodiment, the comparator is configured to use a detection of the rising edge for triggering detection of the falling edge, wherein the detection of the rising edge forms an output of the comparator for causing the rectifying switch to be turned on and a detection of the falling edge forms an output of the comparator for causing the rectifying switch to be turned back off.

Thus, the comparator may be configured to control the rectifying switch to be turned on during a peak of the output voltage signal.

It should be realized that if the rectifying switch is to be turned on based on a minimum of the output voltage signal, the comparator may instead cause the rectifying switch to be turned on based on detecting the falling edge and the comparator may cause the rectifying switch to be turned back off based on detecting the rising edge.

According to an embodiment, the comparator is configured to receive input for controlling a threshold for the detection of the rising edge and a threshold for the detection of the falling edge.

This implies that the comparator may receive control inputs for controlling thresholds for detection of edges such that the comparator may be tuned. The comparator may for instance be tuned such that control signals for controlling the rectifying switch may correspond closely to control signals that would be generated by a synchronous control of the rectifying switch.

According to an embodiment, output of detection of the rising edge and/or output of the detection of the falling edge is configured to provide calibration of the input for controlling the thresholds.

This implies that the comparator may be calibrated. The comparator may comprise separate components for detection of the rising edge and detection of the falling edge, respectively. The output of a component may be used for calibrating that component, such that, for example, the output from the component for detection of the rising edge may be used for calibrating the component for detection of the rising edge. However, it should be realized that the output of one component may be used for calibrating another component, such that, for example, the output from the component for detection of the rising edge may be used for calibrating the component for detection of the falling edge.

According to an embodiment, the charging circuitry is selectively configurable between a first mode and a second mode, wherein: in the first mode, the delay circuit is active and configured to control the state of the comparator such that the comparator is disabled for a delay period between successive instances of the rectifying switch being turned on; and in the second mode, the delay circuit is inactive and the comparator or an additional comparator is configured to continuously identify the extreme point of the output voltage signal of the power receiver and configured to cause the rectifying switch to be turned on based on identification of the extreme point of the output voltage signal.

Thus, the charging circuitry may be selectively set in a first mode or in a second mode such that the charging circuitry may be adapted to conditions for the wireless power transfer.

The comparator used in the second mode may advantageously be a separate comparator from the comparator used in the first mode. The comparator used in the second mode may be a continuous-time comparator.

According to an embodiment, the power receiver comprises an inductor connected to a capacitor, wherein the power receiver is configured to receive power through resonant inductive wireless power transfer.

Inductive wireless power transfer may be advantageously used in various applications of wireless power transfer. Thus, the power receiver comprising an inductor and a capacitor may ensure that the charging circuitry is adapted for use in efficient wireless power transfer.

The inductor and capacitor may form a resonant LC-tank of the power receiver.

According to a second aspect, there is provided an implant device comprising the charging circuitry according to the first aspect, wherein the implant device is configured to be implanted in a body and configured to receive power through wireless power transfer to the charging circuitry.

Effects and features of this second aspect are largely analogous to those described above in connection with the first aspect. Embodiments mentioned in relation to the second aspect are largely compatible with the first aspect.

The charging circuitry may be advantageously used in applications wherein a relatively weak coupling may be formed between a power transmitter and the power receiver. When powering an implant device, the coupling may be weak as the wireless power transfer may need to be made through tissue while a signal strength may need to be limited in order not to cause any damage to tissue through which powering signals are transmitted.

Thus, the charging circuitry may be suitably used in an implant device.

The charging circuitry of the first aspect may alternatively be advantageously used in other applications wherein there is a relatively weak coupling may be formed between a power transmitter and the power receiver. There may for instance be a relatively weak coupling between the power transmitter and the power receiver, when the power transmitter and the power receiver are arranged at a large distance from each other. This may occur when the power receiver is arranged at a remote location or a location which is difficult to access in a convenient manner. The charging circuitry may for instance be arranged in a sensor device or other type of device which is part of a network, wherein devices are distributed in order to provide a distributed sensor function or other type of function. Such devices may for instance be arranged for long-term monitoring of a property and may need to be recharged to ensure long lifetime and low maintenance of the network. The devices may be connected to Internet for providing communication, e.g., of sensor data, over Internet, such that the devices may be Internet of Things (IoT) devices.

According to a third aspect, there is provided a method for receiving power through wireless power transfer, said method comprising: receiving, by a power receiver, an alternating current (AC) wireless power signal and storing energy through resonance by the power receiver; identifying, by a comparator, an extreme point of an output voltage signal of the power receiver; upon identifying the extreme point of the output voltage signal, turning on a rectifying switch for connecting an output of the power receiver to a load; and disabling the comparator for a delay period between successive instances of the rectifying switch being turned on.

Effects and features of this third aspect are largely analogous to those described above in connection with the first and second aspects. Embodiments mentioned in relation to the third aspect are largely compatible with the first and second aspects.

The method allows the rectifying switch to be turned on selectively, such that the rectifying switch need not be turned on during each period of the output voltage signal. Thanks to the disabling of the comparator for a delay period, the power consumption for controlling charging may be limited such that the method allows for efficient wireless power transfer.

It should be realized that the disabling of the comparator may be based on output of a delay circuit. The delay circuit may control a state of the comparator, such that the delay circuit may not necessarily provide a control signal for disabling the comparator. Rather, the comparator may be set to be disabled by default and the delay circuit may provide a control signal to enable the comparator. Hence, disabling of the comparator may be achieved based on absence of an enable signal.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view of a conventional half-wave active rectifier.
Fig. 2 is a schematic view of operational waveforms of the half-wave active rectifier of Fig. 1.
Fig. 3 is a schematic view of a charging circuitry according to a first embodiment.
Fig. 4 is a schematic view of a comparator of the charging circuitry of the first embodiment.
Fig. 5 is a schematic view of a charging circuitry according to a second embodiment.
Fig. 6 is a schematic view of an implant device according to an embodiment.
Fig. 7 is a flow chart of a method according to an embodiment.

### Detailed description

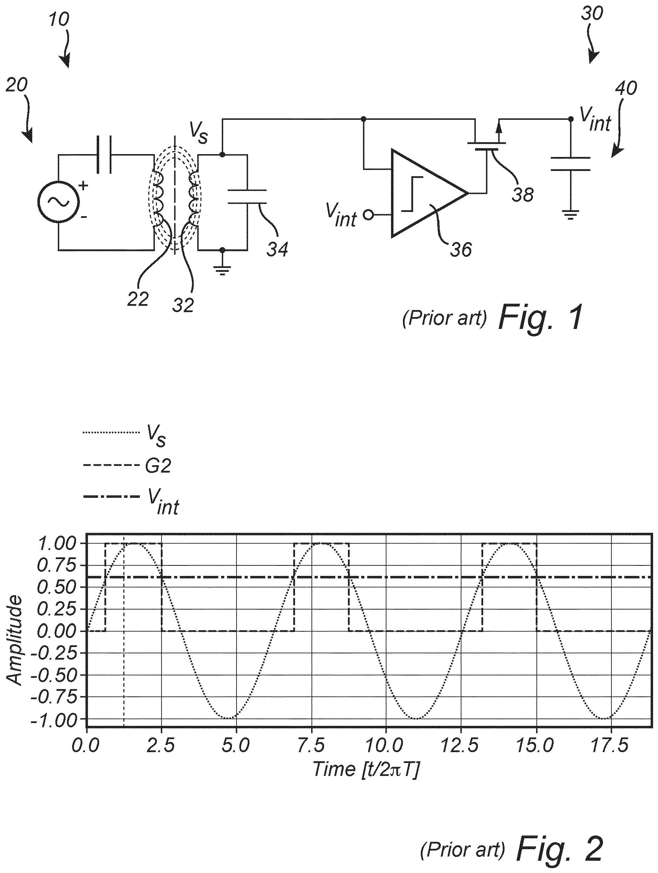

Fig. 1 illustrates a conventional half-wave active rectifier for an inductive wireless power transfer system 10. The power transfer system 10 comprises a power transmitter 20 configured to generate a time-varying magnetic field by applying an alternating current (AC) voltage to a transmitter coil 22. The power transfer system 10 further comprises a power receiver 30 comprising a coil 32 which is connected in parallel to a capacitor 34, forming a resonant LC-tank.

The magnetic field provided by the power transmitter 20 couples to the coil 32 in the power receiver 30. The output voltage signal *vₛ* from the power receiver 30 is provided to a continuous-time comparator 36. The comparator 36 compares the output voltage signal *vₛ* to a load voltage *Vᵢₙₜ.* The comparator 36 outputs a control signal to turn on a rectifying switch 38 when the output voltage signal *vₛ* exceeds the load voltage *Vᵢₙₜ.* Thus, the control of the rectifying switch 38 by the comparator 36 allows current to flow from the LC-tank only when the output voltage signal *vₛ* exceeds the load voltage *Vᵢₙₜ* such that a direct current (DC) voltage is created at the load 40 (schematically illustrated here by a capacitor).

Fig. 2 illustrates operational waveforms of the half-wave rectifier discussed above, showing amplitude as a function of time where T is a carrier period. As shown, the output voltage signal *vₛ* exceeds the load voltage *Vᵢₙₜ* during each period of the output voltage signal *vₛ* such that rectifier operation may be achieved forming a signal from the comparator 36 denoted by G2 in Fig. 2.

If power coupled into the power receiver 30 is reduced (e.g., due to an increased distance to the power transmitter 20 or due to misalignment between the power transmitter 20 and the power receiver 30), an amplitude of the output voltage signal *vₛ* drops such that a margin between the output voltage signal *vₛ* and the load voltage *Vᵢₙₜ* is reduced. This may imply that the power transfer may be relatively inefficient and may be highly dependent on accurate timing of switching of the rectifying switch 38.

The control of the rectifying switch 38 may be alternatively provided by a synchronous control involving a counter for counting a number of periods (cycles) of the output voltage signal *vₛ* such that the LC-tank may resonate for a programmable N number of periods before the rectifying switch 38 is turned on.

It is an insight of the present description that the power consumption of the comparator and logic for providing synchronous control limits efficiency of wireless power transfer.

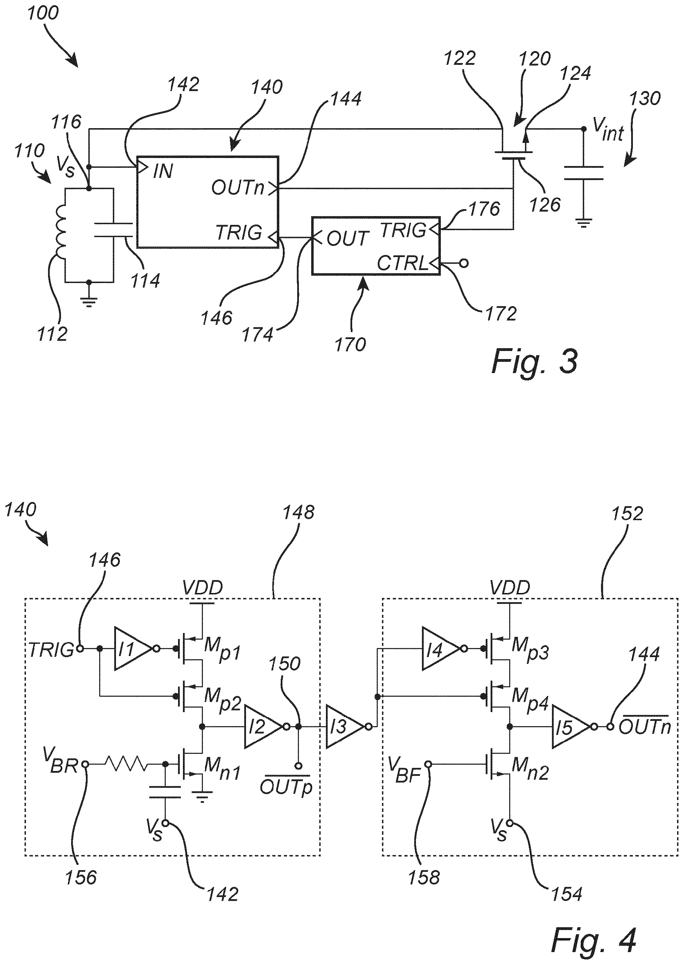

Referring now to Fig. 3, a charging circuitry 100 according to a first embodiment will be described. The charging circuitry 100 comprises a power receiver 110. The power receiver 110 shown in Fig. 3 comprises an inductor 112 connected in parallel with a capacitor 114 forming an LC-tank configured to receive power through resonant inductive wireless power transfer. However, it should be realized that the power receiver 110 may be configured in a different manner for receiving an AC wireless power signal. For instance, the power receiver 110 may be configured for capacitive resonant wireless power transfer.

The power receiver 110 is configured to generate an output voltage signal *vₛ* at an output node 116 of the power receiver 110. The output node 116 may be connected to a rectifying switch 120. The rectifying switch 120 may comprise a first terminal 122 connected to the output node 116 of the power receiver 110 and a second terminal 124 connected to a load 130.

The rectifying switch 120 may be configured to selectively open or close a connection between the first terminal 122 and the second terminal 124 so as to control whether the output node 116 of the power receiver 110 is connected to the load 130.

The rectifying switch 120 may for instance be formed by a transistor, wherein the first terminal 122 and the second terminal 124 form a drain and a source of the transistor. In Fig. 3, the rectifying switch 120 is illustrated as an n-type metal-oxide-semiconductor (NMOS) transistor with the first terminal 122 being a drain and the second terminal being a source of the NMOS transistor. However, it should be realized that the rectifying switch 120 may alternatively be formed as a p-type metal-oxide-semiconductor (PMOS) transistor with the first terminal being a source and the second terminal being a drain of the PMOS transistor. It should also be realized that the rectifying switch 120 need not necessarily be formed by a transistor and that other types of switches known to the person skilled in the art may be used.

The rectifying switch 120 being formed by a transistor further comprises a gate terminal 126. A signal received on the gate terminal 126 may control whether a channel is opened or closed between the first terminal 122 and the second terminal 124 of the transistor. In this manner, the transistor may be configured to operate as a switch.

The charging circuitry 100 further comprises a comparator 140. The comparator 140 is connected to the output node 116 of the power receiver 110 to receive the output voltage signal *vₛ* as an input to an input terminal 142 of the comparator 140.

The comparator 140 may be configured to compare the output voltage signal *vₛ* to a threshold such that the comparator 140 could identify when the output voltage signal *vₛ* exceeds the threshold for determining when the rectifying switch 120 is to be turned on. For instance, the threshold may be based on the load voltage *Vᵢₙₜ.* However, as illustrated in Fig. 3, the comparator 140 may instead be configured to detect an edge of the output voltage signal *vₛ*. The comparator 140 may be configured to perform (nonlinear) integration of the output voltage signal *vₛ* received at the input terminal 142 of the comparator 140. The comparator 140 may be calibrated such that the integration may generate an indication of detection of an edge of the output voltage signal *vₛ* corresponding to the output voltage signal *vₛ* exceeding a threshold. Thus, the comparator 140 may be viewed as being provided with an internal threshold.

The comparator 140 is configured to identify an extreme point of the output voltage signal *vₛ*. Thus, the comparator 140 may be configured to identify a minimum or maximum of the output voltage signal *vₛ* during a period of the output voltage signal *vₛ*. As indicated above, the comparator 140 may be configured to identify the extreme point by identifying that the output voltage signal *vₛ* exceeds a threshold value or by identifying that the output voltage signal *vₛ* changes quickly to form an edge in the output voltage signal *vₛ*. It should be realized that the rectifying switch 120 is to be turned on during a period around the extreme point, i.e., during a period when the output voltage signal *vₛ* exceeds the load voltage *Vᵢₙₜ.* Thus, the comparator 140 need not determine an absolute maximum or minimum of the output voltage signal *vₛ* but may rather identify a duration during which the output voltage signal *vₛ* is sufficiently large (or small) such that the rectifying switch 120 should be turned on.

The comparator 140 may be configured to cause the rectifying switch 120 to be turned on. The comparator 140 may thus provide an output based on identifying the extreme point of the output voltage signal *vₛ*. The comparator 140 may provide an activation signal at an output node 144. The output terminal 144 may be directly connected to the gate terminal 126 of the rectifying switch 120. Thus, the rectifying switch 120 may be controlled by the activation signal. For instance, the rectifying switch 120 may be controlled such that the rectifying switch 120 is turned on to close a connection between the first terminal 122 to the second terminal 124 when the activation signal is high and the rectifying switch 120 is turned off to open a connection between the first terminal 122 to the second terminal 124 when the activation signal is low.

The comparator 140 may be configured to provide an activation signal that is high (or low) during a period when the rectifying switch 120 is to be turned on. Thus, the rectifying switch 120 may be controlled to be on or off based on a state of the activation signal.

The comparator 140 may be configured to provide an activation signal that is synchronized with the output voltage signal *vₛ*. However, the comparator 140 may be selectively disabled based on a signal from a delay circuit 170. The delay circuit 170 may operate independently of the output voltage signal *vₛ*. This implies that the charging circuitry 100 may be semi-synchronous. The comparator 140 may be configured to consume power dynamically based on the comparator 140 being selectively enabled when it is desired that the rectifying switch 120 is to be turned on. For instance, the delay circuit 170 may be set such that the comparator 140 is only disabled during N-1 periods of the output voltage signal *vₛ*. This implies that the power consumption of the comparator 140 may scale down with N, being the number of periods (or resonance cycles) of the output voltage signal *vₛ* during which the power receiver 110 should store energy.

The delay circuit 170 may be configured to control a state of the comparator 140 for ensuring that the comparator 140 is disabled for a delay period so as to limit power consumption of the comparator 140. The delay circuit 170 may be configured to provide an enable signal which activates the comparator 140 such that the comparator 140 is disabled unless activated by the delay circuit 170. Alternatively, the delay circuit 170 may be configured to provide a disable signal which deactivates the comparator 140.

The delay circuit 170 may be implemented in various manners as known to the person skilled in the art. The delay circuit 170 may comprise a plurality of delay elements, wherein a signal is propagated through the delay elements. The delay circuit 170 may be configured to receive an external control signal at an input terminal 172 for controlling the delay circuit 170. For instance, the control signal may be used for controlling a number of delay elements through which a signal is to be propagated for controlling the delay provided by the delay circuit 170. However, it should be realized that the delay circuit 170 may alternatively be statically arranged so as to provide a static, constant delay.

The delay circuit 170 may be configured to ensure that the comparator 140 is disabled for a delay period between successive instances of the rectifying switch 120 being turned on. Thus, the comparator 140 may be disabled during a plurality of periods of the output voltage signal *vₛ* allowing an amplitude of the periods of the output voltage signal *vₛ* to increase by being accumulated over the plurality of periods. This implies that, when the rectifying switch 120 is turned on, the output voltage signal *vₛ* may exceed the load voltage *Vᵢₙₜ* by a margin.

It should be realized that the delay circuit 170 need not provide a delay that exactly matches a number of periods of the output voltage signal *vₛ*. On the contrary, once the comparator 140 is enabled after the delay period has passed, the comparator 140 will identify a next extreme point in the output voltage signal *vₛ* such that the rectifying switch 120 may be turned on in synchronization with a value of the output voltage signal *vₛ* being around a desired extreme point.

Further, it should be realized that the delay circuit 170 need not necessarily provide a delay that exceeds a single period of the output voltage signal *vₛ*. Rather, the delay circuit 170 may control the comparator 140 to be disabled for a fraction of a period of the output voltage signal *vₛ*. This may still allow power consumption of the comparator 140 to be limited.

There may be an optimum delay of the delay circuit 170 for minimizing power consumption in relation to the power received by the charging circuitry 100. The delay period may be set to correspond to an optimum number of periods of the output voltage signal *vₛ*, wherein the number of periods should increase as long as an increase in energy extracted from the LC-tank due to increased energy stored in the LC-tank during an additional resonance period is larger than energy lost in the LC-tank and due to static power of control circuitry (comparator 140, delay circuit 170) during the additional resonance period.

For a non-ideal inductor 112, an optimum delay may be defined. The optimum delay may be a function of power received, a quality factor of the LC-tank, power efficiency as a function of energy stored in the LC-tank and static and dynamic power consumption of the control circuitry.

For instance, the delay circuit 170 may be configured to provide a delay for a delay period corresponding to approximately 10 periods of the output voltage signal *vₛ*. However, with a very high quality factor (e.g., quality factor Q > 100) of the LC-tank, a delay period corresponding to 20 periods of the output voltage signal *vₛ* may be used.

The delay circuit 170 may be configured to provide a delay for a delay period. The delay circuit 170 may be configured to output a trigger at an output terminal 174. The trigger may control the state of the comparator 140. For instance, the trigger may provide an enable signal to the comparator 140 such that the comparator 140 is activated.

The comparator 140 may comprise a trigger terminal 146 connected to the output terminal 174 of the delay circuit 170. Thus, the comparator 140 may be configured to be activated based on receiving a trigger signal at the trigger terminal 146. The comparator 140 may thus start to analyze the output voltage signal *vₛ* for finding an extreme point. In other words, the comparator 140 may initiate identifying of the extreme point of the output voltage signal Vs.

The comparator 140 may provide an activation signal at an output node 144 and the rectifying switch 120 may be controlled by the activation signal. As shown in Fig. 3, the activation signal may also be received by the delay circuit 170 at a trigger terminal 176 of the delay circuit 170. Thus, start of the delay period provided by the delay circuit 170 may be set by the activation signal. The delay circuit 170 may be configured to start a delay period based on the activation signal going high (to turn on the rectifying switch 120) or based on the activation signal going low (to turn off the rectifying switch 120 after the rectifying switch 120 has been turned on).

It should be further realized that the rectifying switch 120 and the delay circuit 170 need not receive signals at the gate terminal 126 and the trigger terminal 176 directly from the comparator 140. Also, the gate terminal 126 and the trigger terminal 176 need not receive exactly the same signal. Rather, there may be additional component(s) between the comparator 140 and one of the gate terminal 126 and the trigger terminal 176.

Referring now to Fig. 4, the comparator 140 according to one embodiment is shown in detail. As illustrated in Fig. 4, the comparator 140 may comprise a first component 148 configured to detect a rising edge of the output voltage signal *vₛ*. The output voltage signal *vₛ* is received as input to the comparator 140 at the input terminals 142 and 152 of the comparator 140. Even though the output voltage signal *vₛ*, from perspective of the comparator 140, is an input signal, the signal will still be referred to as output voltage signal *vₛ* in order to use a consistent name of the signal.

The first component 148 is configured to provide an output signal *O̅U̅T̅p̅* at an output 150. The first component 148 may be enabled based on receiving an enable signal (trigger) from the delay circuit 170 at the trigger terminal 146. In Fig. 4, the trigger constitutes a change from high to low input at the trigger terminal 146.

The output signal *O̅U̅T̅p̅* may be configured to go high when a rising edge is detected in the output voltage signal *vₛ* at the input terminal 142, after the comparator 140 has received a suitable trigger at the trigger terminal 146. The output 150 may further be connected to a second component 152 configured to detect a falling edge of the output voltage signal *vₛ*. The second component 152 is triggered by the detection of the rising edge (the output signal *O̅U̅T̅p̅* going high).

The second component 152 is configured to provide an output signal *O̅U̅T̅n̅* at a node 144. The output signal *O̅U̅T̅n̅* may be provided at the output terminal 144 of the comparator. The output signal *O̅U̅T̅n̅* may change from high to low upon the second component 152 being triggered by the output signal *O̅U̅T̅p̅.* Thus, the comparator 140 may provide an activation signal on the output terminal 144 that goes low when a rising edge is detected in the output voltage signal *vₛ*.

The second component 152 also receives the output voltage signal *vₛ* at an input terminal 154. The second component 152 is configured to detect a falling edge of the output voltage signal *vₛ* such that the output signal *O̅U̅T̅n̅* goes high when the falling edge is detected.

Thus, the comparator 140 may be configured to output an activation signal at output terminal 144 which is only low starting at detection of the rising edge and ending at detection of the falling edge and high otherwise. This implies that the comparator 140 may provide an activation signal such that detection of the rising edge causes the rectifying switch 120 to be turned on and detection of the falling edge causes the rectifying switch 120 to be turned back off.

The first component 148 and the second component 152 may each be configured to receive input for controlling a threshold for detection of the rising edge and for controlling a threshold for detection of the falling edge, respectively. Thus, the first component 148 may comprise a control terminal 156 for receiving a bias voltage *V_{BR}* for controlling detection of the rising edge. Similarly, the second component 152 may comprise a control terminal 158 for receiving a bias voltage *V_{BF}* for controlling detection of the falling edge.

It should be realized that the comparator 140 may be modified such that bias currents are provided instead of bias voltages.

The inputs for controlling thresholds of the comparator 140 may be used for tuning the comparator 140 such that the charging circuitry 100 may produce an activation signal that closely matches a signal generated by a synchronous rectifier.

The output signal *O̅U̅T̅p̅* at the output 150 and/or the output signal *O̅U̅T̅n̅* at the output terminal 144 may be used for providing calibration of the inputs for controlling thresholds of the comparator 140. Thus, the output 150 may be used merely for calibration purposes and the comparator 140 may not necessarily be provided with a separate output 150 from the detection of the rising edge.

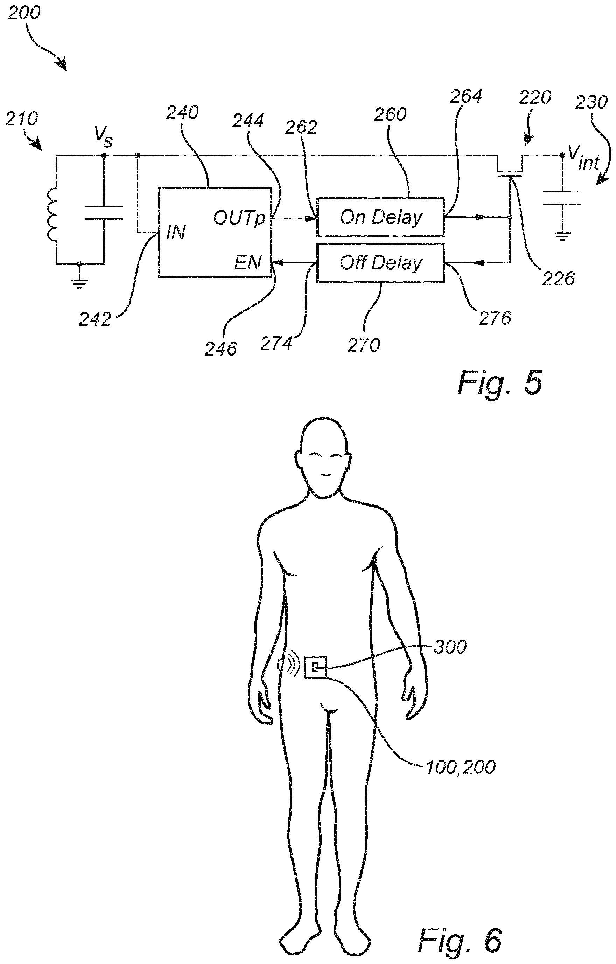

Referring now to Fig. 5, a charging circuitry 200 according to a second embodiment will be described. For brevity, only differences in relation to the charging circuitry 100 according to the first embodiment are discussed below and features that are common to both embodiments are not discussed again here.

The charging circuitry 200 comprises a power receiver 210 which may be identical to the power receiver 110. Also, the charging circuitry comprises a rectifying switch 220 for selectively connecting an output node 216 of the power receiver 210 to a load 230. The rectifying switch 220 may be identical to the rectifying switch 120.

The charging circuitry 200 comprises a comparator 240. Like the comparator 140, the comparator 240 may be configured to receive the output voltage signal *vₛ* from the power receiver 210 at an input terminal 242. However, the comparator 240 may be configured to only detect one edge of the output voltage signal *vₛ*. For instance, if a peak of the output voltage signal *vₛ* is to be detected, the comparator 240 may only be configured to detect a rising edge (i.e., an edge preceding the peak). Thus, the comparator 240 may be implemented based only on the first component 148 of the comparator 140 described above.

The comparator 240 may thus provide an output signal *OUTp* at the output terminal 244 which is based on detection of a rising edge. The output signal *OUTp* may thus provide an indication when the rectifying switch 220 is to be turned on. However, the output signal *OUTp* may not provide any indication of when the rectifying switch 220 is to be turned off.

The output signal *OUTp* may be provided to an on-delay circuit 260. The on-delay circuit 260 may receive the output signal *OUTp* from the comparator 240 at an input 262. The on-delay circuit 260 may be configured to provide an output signal at an output 264 which is connected to the gate terminal 226 of the rectifying switch 220. The on-delay circuit 260 may provide an output signal that goes high upon *OUTp* provided at the input 262 going high. The on-delay circuit 260 may further be programmed to provide a correct on-time for the rectifying switch 220 such that the output signal is maintained high during a desired on-time.

The on-delay circuit 260 may further be connected to a trigger terminal 276 of the delay circuit 270. The delay circuit 270 may correspond to the delay circuit 170 of the charging circuitry 100 of the first embodiment. The delay circuit 270 may thus be considered an off-delay circuit 270 such that the off-delay circuit 270 controls an off-time of the comparator 240 and the on-delay circuit 260 controls an on-time of the rectifying switch 220.

The off-delay circuit 270 may thus be configured to provide a delay for a delay period. The off-delay circuit 270 may be configured to output a trigger at the output terminal 274 after the delay period has passed. The trigger may control the state of the comparator 240. For instance, the trigger may provide an enable signal to the comparator 240 at the trigger terminal 246 such that the comparator 240 is activated.

As shown in the charging circuitry 200 of Fig. 5, start of the delay period provided by the delay circuit 270 may be set by a signal which is not received directly from the comparator 240.

It should be further realized that the charging circuitry of any of the above embodiments may further be configured to be selectively set in a first mode and a second mode.

The charging circuitry may for instance be selectively set in the first mode when a weak powering signal is received by the power receiver. Thus, when there is a need for ensuring that power consumption of the charging circuitry is limited the charging circuitry may be set in the first mode.

In the first mode, the charging circuitry may operate as described above for the first and second embodiments of the charging circuitry. Thus, the delay circuit may be active, and the comparator may be controlled to be disabled for a delay period in order to reduce power consumption.

The charging circuitry may for instance be selectively set in the second mode when a strong powering signal is received. In such case, there may not be a need to disable the comparator so as to accumulate energy during plural resonance cycles of the power receiver. Therefore, the limited power consumption provided in the embodiments of the charging circuitry discussed above may not be as crucial.

In the second mode, function of the delay circuit may not be needed, and the delay circuit may be inactive. Further, the comparator described above, or a different comparator of the charging circuitry may be configured to continuously identify the extreme point of the output voltage signal of the power receiver. For instance, a continuous-time comparator may be used.

The comparator may thus, in the second mode, be configured to cause the rectifying switch to be turned on during each period of the output voltage signal based on identification of the extreme point of the output voltage signal.

Referring now to Fig. 6, the charging circuitry 100, 200 according to the first or second embodiment may be advantageously provided in an implant device 300. Although it should be realized that the charging circuitry 100, 200 may be part of any device for use in any wireless power transfer regardless of where the device to be powered is arranged in relation to a power transmitter, the charging circuitry 100, 200 is particularly suited for use in the implant device 300.

The implant device 300 may be configured to be implanted in a human body or an animal body and may need wireless power transfer for powering the implant device 300 while being implanted. This implies that a relation between the implant device 200 and the power transmitter may not be known or controlled. The wireless power transfer may be subject to dynamically changing conditions, for instance due to implant device 300 and the power transmitter being subject to relative movement and/or orientation of the implant device 300 in relation to the power transmitter being changed. This implies that a weak powering signal may be received by the charging circuitry 100, 200 such that the charging circuitry 100, 200 may need to receive wireless power transfer in an efficient manner.

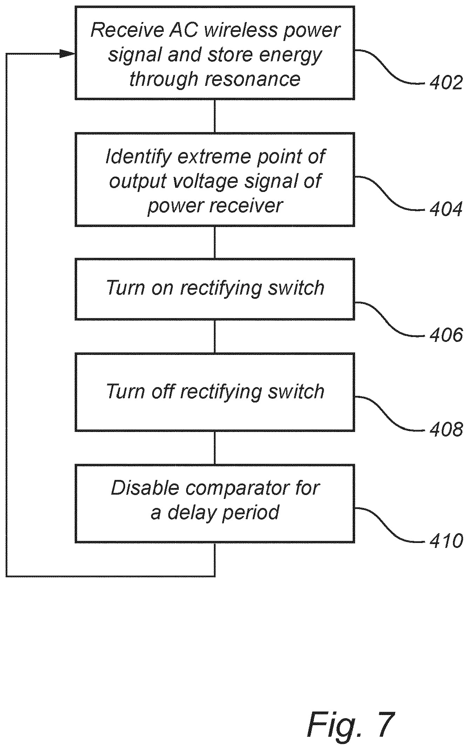

Referring now to Fig. 7, a method for receiving power through wireless power transfer will be described.

The method comprises receiving 402, by a power receiver, an AC wireless power signal and storing energy through resonance by the power receiver.

The method further comprises identifying 404, by a comparator, an extreme point of an output voltage signal of the power receiver. The method further comprises upon identifying the extreme point of the output voltage signal, turning on 406 a rectifying switch for connecting an output of the power receiver to a load. Thus, the identifying may ensure that the output voltage signal is at an appropriate level for the rectifying switch to be turned on and power being transferred to the load. The rectifying switch may be turned off 408 again in relation to a level of the output voltage signal dropping.

The method further comprises disabling 410 the comparator for a delay period between successive instances of the rectifying switch being turned on. This implies that a delay period is set during which the comparator may be disabled. Thus, the comparator does not consume power during the delay period or consumes a very small amount of power during the delay period. This implies that energy is allowed to be accumulated by the power receiver and then transferred to the load while energy consumed by a charging circuitry is minimized or at least limited during accumulation of energy by the power receiver.

After the delay period, the method may return again to the receiving 402.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A charging circuitry (100; 200) for receiving power through wireless power transfer, said charging circuitry (100: 200) comprising:
a power receiver (110; 210) configured to receive an alternating current, AC, wireless power signal and configured to store energy through resonance;
a rectifying switch (120; 220) configured to selectively connect an output (116; 216) of the power receiver (110; 210) to a load (130; 230);
a comparator (140; 240) connected to the output (116; 216) of the power receiver (110; 210) and configured to identify an extreme point of an output voltage signal (*vₛ*) of the power receiver (110; 210) and configured to cause the rectifying switch (120; 220) to be turned on based on identification of the extreme point of the output voltage signal (*vₛ*); and
a delay circuit (170; 270) configured to control a state of the comparator such that the comparator (140; 240) is disabled for a delay period between successive instances of the rectifying switch (120; 220) being turned on.

2. The charging circuitry according to claim 1, wherein the delay circuit (170; 270) is configured to output a trigger for activating the comparator (140; 240), wherein the comparator (140; 240) is configured to, upon receiving the trigger, initiate identifying of the extreme point of the output voltage signal (*vₛ*) of the power receiver (110; 210).

3. The charging circuitry according to claim 1 or 2, wherein the delay circuit (170; 270) is configured to receive an external control signal for controlling the delay period of the delay circuit (170; 270).

4. The charging circuitry according to any one of the preceding claims,
wherein the comparator (140; 240) is configured to output an activation signal for turning on the rectifying switch (120; 200) upon the identification of the extreme point of the output voltage signal (*vₛ*) of the power receiver (110; 210), wherein the delay circuit (170; 270) is also configured to receive the activation signal for triggering start of a following delay period.

5. The charging circuitry according to any one of the preceding claims,
wherein the comparator (140; 240) is configured to detect at least one edge of the output voltage signal (*vₛ*).

6. The charging circuitry according to claim 5, wherein the comparator (140; 240) is configured to detect a rising edge and a falling edge of the output voltage signal (*vₛ*).

7. The charging circuitry according to claim 6, wherein the comparator (140; 240) is configured to use a detection of the rising edge for triggering detection of the falling edge, wherein the detection of the rising edge forms an output of the comparator (140; 240) for causing the rectifying switch (120; 220) to be turned on and a detection of the falling edge forms an output of the comparator (140; 240) for causing the rectifying switch (120; 220) to be turned back off.

8. The charging circuitry according to claim 7, wherein the comparator (140; 240) is configured to receive input for controlling a threshold for the detection of the rising edge and a threshold for the detection of the falling edge.

9. The charging circuitry according to claim 8, wherein output of detection of the rising edge and/or output of the detection of the falling edge is configured to provide calibration of the input for controlling the thresholds.

10. The charging circuitry according to any one of the preceding claims,
wherein the charging circuitry (100; 200) is selectively configurable between a first mode and a second mode, wherein:
in the first mode, the delay circuit (170; 270) is active and configured to control the state of the comparator (140; 240) such that the comparator (140; 240) is disabled for a delay period between successive instances of the rectifying switch (120; 220) being turned on; and
in the second mode, the delay circuit (170; 270) is inactive and the comparator (140; 240) or an additional comparator is configured to continuously identify the extreme point of the output voltage signal (*vₛ*) of the power receiver (110; 210) and configured to cause the rectifying switch (120; 220) to be turned on based on identification of the extreme point of the output voltage signal (*vₛ*).

11. The charging circuitry according to any one of the preceding claims,
wherein the power receiver (110; 210) comprises an inductor (112) connected to a capacitor (114), wherein the power receiver (110; 210) is configured to receive power through resonant inductive wireless power transfer.

12. An implant device (300) comprising the charging circuitry (100; 200) according to any one of the preceding claims, wherein the implant device (300) is configured to be implanted in a body and configured to receive power through wireless power transfer to the charging circuitry (100; 200).

13. A method for receiving power through wireless power transfer, said method comprising:
receiving (402), by a power receiver, an alternating current, AC, wireless power signal and storing energy through resonance by the power receiver;
identifying (404), by a comparator, an extreme point of an output voltage signal of the power receiver;
upon identifying the extreme point of the output voltage signal, turning on (406) a rectifying switch for connecting an output of the power receiver to a load; and
disabling (410) the comparator for a delay period between successive instances of the rectifying switch being turned on.
